# EUROPEAN PATENT APPLICATION

(11) **EP 4 560 018 A1**
(43) Date of publication of application: **28.05.2025**
(21) Application number: 23383196.5
(22) Date of filing: 22.11.2023
(51) Int. Cl.: C12N 15/10, C12Q 1/6806

(54) **METHOD FOR THE GENERATION OF A CDNA LIBRARY FROM AN RNA SAMPLE**

(71) Applicant: Servicio Andaluz de Salud, 41071 Sevilla (ES); Universidad de Granada, 18071 Granada (ES)
(72) Inventor: CARAZO GALLEGO, Ángel, 18012 Granada (ES); REDRUELLO ROMERO, Anaïs, 18012 Granada (ES); LEÓN LÓPEZ, Josefa, 18012 Granada (ES); MORALES SANTANA, Sonia, 18012 Granada (ES)
(74) Representative: Hoffmann Eitle

(57) **Abstract**

The present invention refers to the medical field. Particularly, the present invention refers to a method for the generation of a cDNA library from an RNA sample, which comprises: a) providing an adenylated the RNA, b) hybridizing the adenylated RNA with a first synthetic oligonucleotide that comprises a poly T tail, c) reverse-transcribing the hybridized RNA to cDNA, d) attaching the cDNA strand to a functionalized particle through a chemical bond, e) dehybridizing the RNA to expose the cDNA, characterized in that the steps (b) and/or (c) are performed in solution, and in that the method does not comprise ligation reactions between oligonucleotides. This methodology has multiple applications, including miRNA sequencing and quantification.

## Description

### FIELD OF THE INVENTION

The present invention refers to the molecular biology field. Particularly, the present invention refers to a method for the generation of a cDNA library from an RNA sample, which comprises: a) providing an adenylated RNA, b) hybridizing the adenylated RNA with a first synthetic oligonucleotide that comprises a poly T tail, c) reverse-transcribing the hybridized RNA to cDNA, d) attaching the cDNA strand to a functionalized particle through a chemical bond, e) dehybridizing the RNA to expose the cDNA, characterized in that the steps (b) and/or (c) are performed in solution, and in that the method does not comprise ligation reactions between oligonucleotides. This methodology has multiple applications, including miRNA sequencing and quantification.

### STATE OF THE ART

Eukaryotic cells (and some viruses) produce small non-coding RNA molecules called microRNA (miRNA) that regulate the expression of numerous genes. Their dysregulation is associated with most human chronic pathological processes, including cancer, type 2 diabetes, neurodegenerative diseases and immune disorders.

The pathophysiological relevance of miRNAs, their chemical stability and abundant presence in exosomes (intercellular communication structures) make miRNAs ideal candidates for the search for biomarkers of presence, grade and prognosis of disease, both in solid tissues and in liquid biopsy.

Next-generation sequencing (NGS) is the only methodology capable of accurately quantifying the set of miRNA variants in a biological sample. Contrary to other methods, it enables the identification of new and unknown variants of miRNA and other families of small regulatory RNA (e.g. siRNA or piRNA). However, the methods developed for conventional mass sequencing fail when applied to miRNA chemical characteristics.

The construction of a miRNA library is an essential preliminary step to the actual NGS. It is at this stage where relevant biases are concentrated. Sequencing libraries are made by adding specific DNA sequences (adapters) to the ends of the population of molecules to be sequenced. The adapter sequence, typically 20-60 nucleotides long, depends on the type of sequencing platform.

Current methods of miRNA-seq are based on ligation reactions (of RNA and/or DNA) to add the sequencing adapters. The probability of joining two nucleic acid molecules through a ligation reaction depends on the sequences at their ends. This is referred to as ligation bias.

One of the main characteristics of ligation bias is that it increases as the size of the molecules to be joined decreases. It is a known and accepted issue in conventional NGS, but in the case of miRNA-seq, it becomes exceptionally significant. As a result, some variants are overestimated, while others are underestimated. Additionally, there are ligase-resistant variants that are challenging to detect using miRNA-seq. Currently, several commercial methods are available to perform NGS of small RNAs. However, significant differences have been described in terms of reproducibility, specificity, and sensitivity. The relevance of ligation bias and the disparity between different NGS library preparation methods are reflected in a loss of reproducibility and contradictory results.

cDNA libraries derived from miRNA samples are often wrongfully assumed to be quantitatively and molecularly similar to the original RNA input. However, biases (such as the above-mentioned ligation bias) and artifacts confound the resulting cDNA mixture. In this regard, improving the efficacy of the library preparation procedures could be beneficial to reduce the biases of the method.

Another problem is the amount of resources spent in the preparation of miRNA libraries. These protocols are tedious and costly. Improving the efficiency of currently available methods could also serve a steppingstone for the development of more cost-efficient and time- methodologies.

In the past years, there has been an enormous interest in developing new concepts to reduce biases in massive miRNA sequencing.

In this context, there is a long-felt need to develop an unbiased methods for the generation of cDNA libraries with an improved efficiency compared to currently available unbiased methods, which may be applied not only to long RNA molecules, but also to small RNA (molecules of less than 200 bp) such as miRNA.

The present invention is focused on solving this problem, and a methodology for the generation of cDNA libraries from an RNA sample is herein provided.

### DESCRIPTION OF THE INVENTION

### Brief description of the invention

As explained above, the present invention refers to a method for the generation of a cDNA library from an RNA sample, which comprises: a) providing an adenylated RNA, b) hybridizing the adenylated RNA with a first synthetic oligonucleotide that comprises a poly T tail, c) reverse-transcribing the hybridized RNA to cDNA, d) attaching the cDNA strand to a functionalized particle through a chemical bond, e) dehybridizing the RNA to expose the cDNA, characterized in that the steps (b) and/or (c) are performed in solution, and in that the method does not comprise ligation reactions between oligonucleotides. This methodology has multiple applications, including miRNA sequencing and quantification.

Methods currently used for the generation of cDNA libraries from an RNA sample typically comprise, among other steps:
i) hybridizing an adenylated RNA with a synthetic oligonucleotide that comprises a poly T tail, and
ii) reverse-transcribing the hybridized RNA to cDNA using a retro transcriptase enzyme.

In currently available unbiased methods, these two steps may be performed on the surface of a particle that facilitates the capture of the oligonucleotides by centrifugation or magnetic sedimentation (in the case or magnetic particles).

The inventors of the present invention identified that performing these steps with the nucleic acids attached to a surface inevitably decreases the efficiency of the reactions, which can in turn result in biases that lead to the overrepresentation or underrepresentation of specific miRNAs in the cDNA pool. Regarding step i), if the particle is negatively charged, it may hinder the interaction between the dissolved synthetic oligonucleotides and the adenylated RNA attached to the surface of a particle. Regarding step ii) the surface of certain particles can partially inhibit some enzymes (such as the retrotranscriptase).

Consequently, the inventors herein provide a methodology in which these critical steps are performed in solution, rather than on a surface. As a result, the hybridization and retrotranscription reactions are much more efficient. Using synthetic standards, the inventors have verified that performing the first two steps in dilution - rather that with the oligonucleotides attached to the surface of a particle - improves the sensitivity threshold by several orders of magnitude (below 0.01 nM).

As a proof of concept, the inventors of the present invention have demonstrated the successful generation of a cDNA library departing from synthetic miRNA using the procedure described in **Example 2.**

### The proof-of-concept procedure described in Example 2 consists of the following steps:

### Selection of the covalent bond that will be used

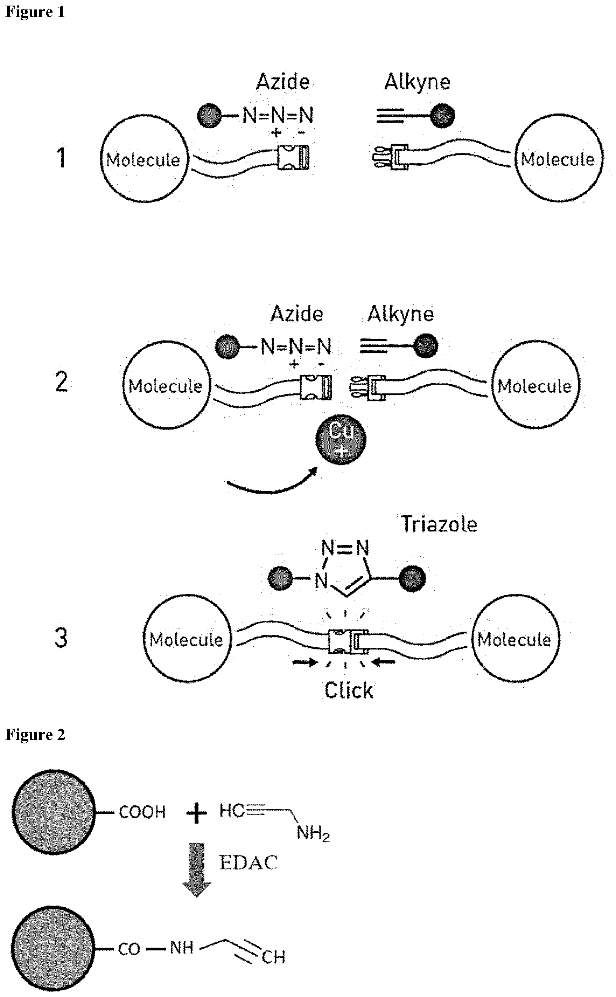

The proposed methodology involves the selection of a covalent bond that will be used to attach particles to oligonucleotides. The present example uses click chemistry (CC) reactions. These reactions produce a triazole bond between an azide and an alkyne group **(****Figure 1****).** CC reactions use Cu (I) as a catalyst. However, there is also a copper-free CC variant that could be used to avoid copper toxicity in biological contexts. The most commonly used cooper-free CC variants originate between a phenyl azide and a cyclooctyne (which has a triple bond within the ring). The reaction product links both groups (covalently) by a mixture of regioisomeric triazoles. Other covalent bonds, such as amide bonds may be used for the same purpose.

### Selection of the particle that will be used

The proposed methodology also involves the selection of a particle. The present example uses magnetic particles, although other particles - such as agar particles - may be used for the same purpose. Some of the requirements that must meet the particle are:
- The particle must not inhibit the activity of the enzymes used in the process. In this example the enzymes used were: terminal deoxynucleotidyl transferase (TdT) and Taq polymerase).
- The particle must have low aggregation capacity and a low sedimentation velocity (in gravity).
- The particle must have a very low or non-existent non-specific affinity for nucleic acids.
- The particle must be stable in a pH of between 5 and 10. In this example the particles had to be stable in a 200 mM NaoH solution, since this solution was used in one of the steps.
- The particle must be able to withstand the denaturation step of PCR cycles, which is generally performed at 95 °C. Thus, it must be stable at high temperatures (up to 95 °C).
- The particle must micrometric or nanometric. The optimal choice would be to work with very small particles (10-100 nm) since reducing the diameter increases the total working surface.

### Functionalization of the particles

The present example has been performed with commercial particles that are functionalized with a carboxylic group. These particles had to be adapted to the CC system, meaning that they had to be functionalized with an azide or an alkyne group. In the present example particles were incubated with propargylamine (a linker that presents an amino group in one end, which can interact with the carboxylic group present in the initial particle, and an alkyne group in the other end) in the presence of EDAC. As a result, the inventors obtained magnetic particles that were functionalized with an alkyne group **(****Figure 2****).**

Particles with carboxylic groups can also be adapted to the CC system using 3-azido propylamine as a linker (also in the presence of EDAC). In this case, the particles will be functionalized with an azide - rather than an alkyne - group.

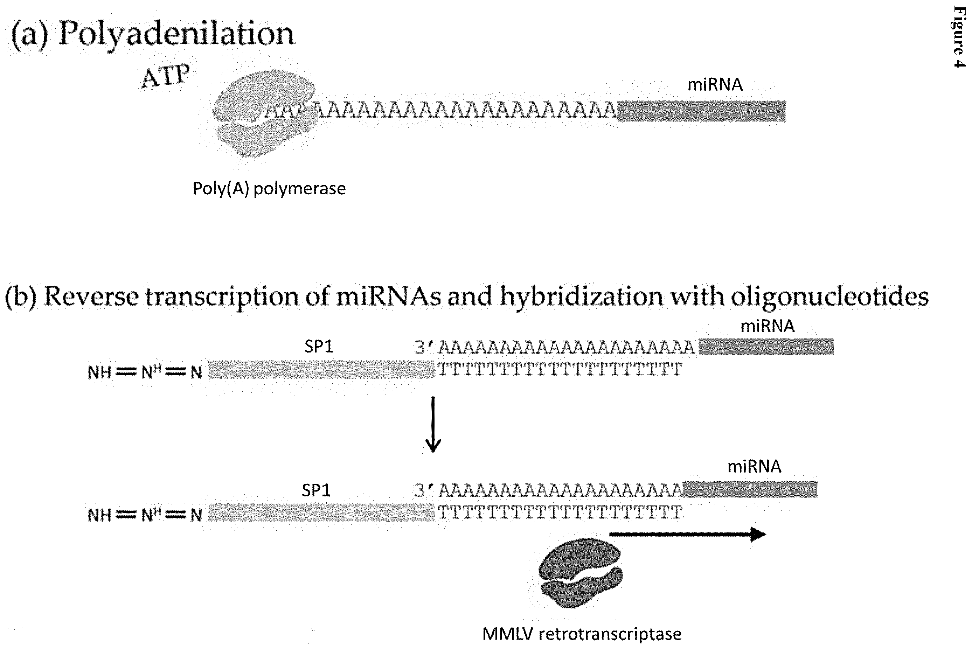

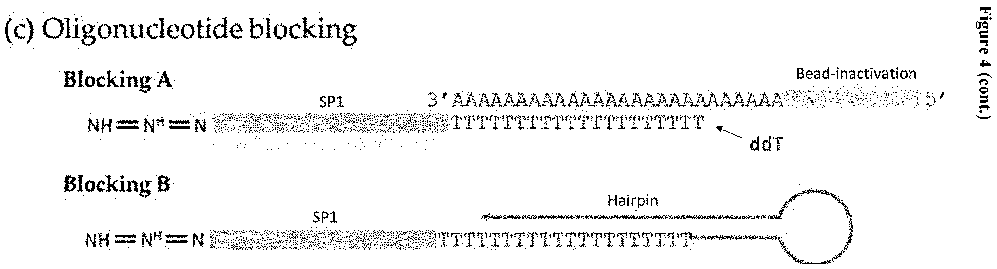

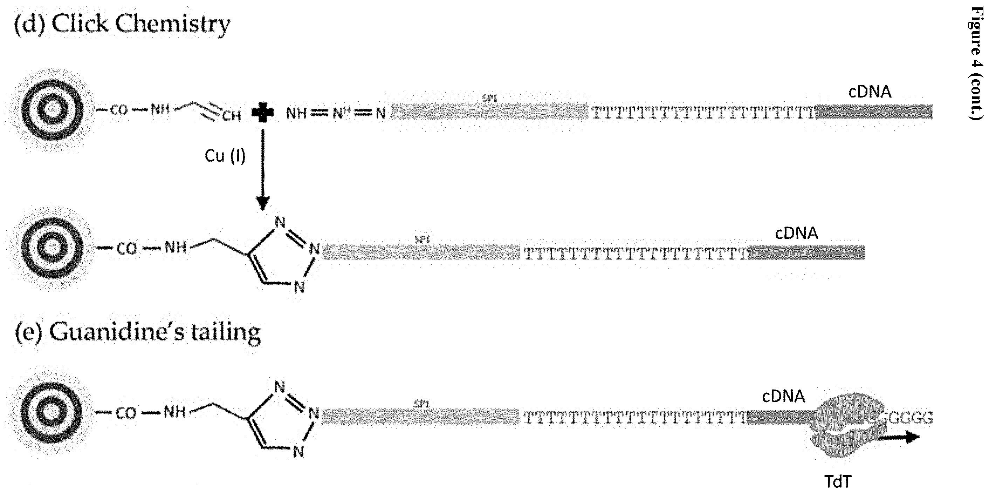

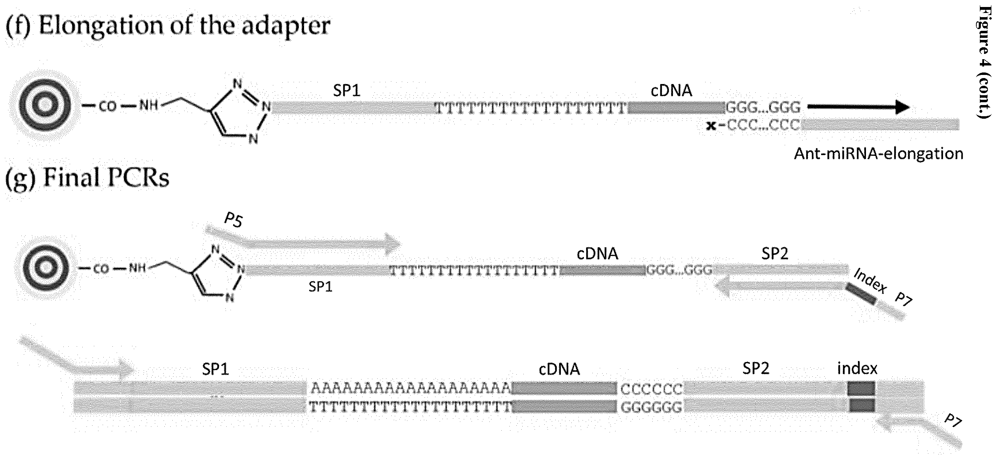

### Generation of the cDNA library (Figure 4)

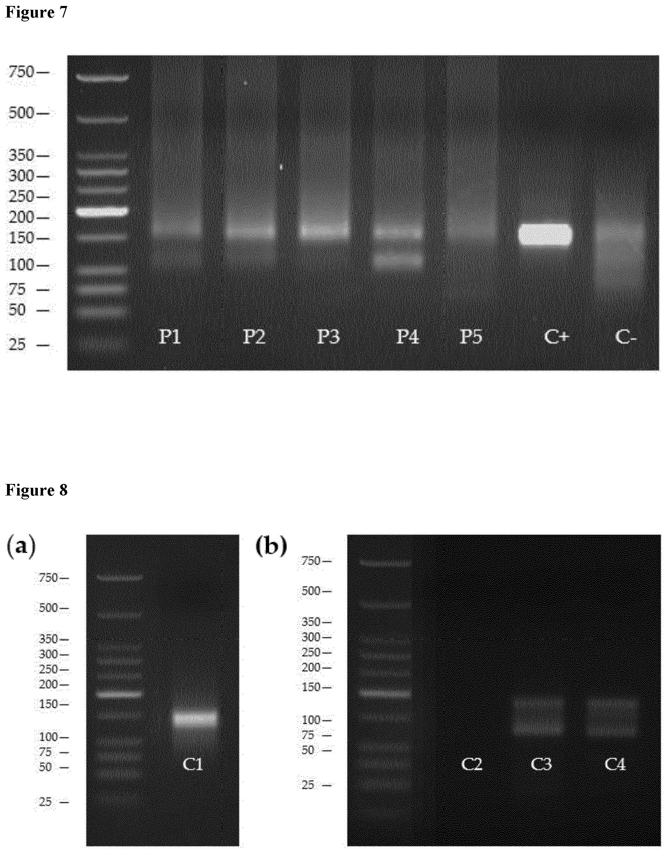

1. Adenylating the 3' end of an RNA using a poly(A) polymerase **(****Figure 4A****).** As a proof of concept, the inventors have used a synthetic miRNA, although this process could be applied to any RNA, including small RNAs. This step is not required if the RNA sample already contains a poly(A) tail at its 3' end, which is the case for mRNA.
2. Capturing the RNAs of interest in solution by hybridization with a synthetic oligonucleotide that comprises: a poly(T) at its 3' end, that is added to capture the RNAs through their poly(A) tail; the sequence of one of the adapters necessary for massive sequencing; and an azide group at the 5' end that will be able to perform a CC reaction with the alkyne group of the particles in later steps **(****Figure 4B****).** It is important to note that the adapter is not added through a ligation reaction, thereby eliminating the ligation bias associated to other methods used for the generation of cDNA libraries.
3. Retrotranscribing the RNAs in solution to obtain cDNA **(****Figure 4B****).**
4. Blocking the oligonucleotides that have not acquired a cDNA sequence in solution by adding ddTTPs that prevent their further extension **(****Figure 4C****, blocking A),** or by introducing a sequence in the oligonucleotide designed to hybridize 3' end of the oligonucleotide in a hairpin structure **(Figure 7C, blocking B)**. The goal in this step is to eliminate, inactivate, or prevent the elongation of the unhybridized synthetic oligonucleotide. In the present example, this was performed using the above methods, but it can also be achieved by eliminating biotinylated oligonucleotides using streptavidin-associated particles.
   It should be noted that steps 1-4 are carried out with the same reaction buffer but changing the enzyme to be used and other components necessary for the reaction. Also, the enzymes are inhibited before the next process begins. Poly(A) polymerase is inhibited with EDTA and retrotranscriptase with heat.
5. Performing the CC reaction, in the presence of copper (I), in which the azide group of the oligonucleotides of step 2 reacts with the alkyne group of the functionalized particles **(****Figure 4D****).** These oligonucleotides will be a mixture of those that have previously captured miRNAs, and already have them in cDNA form, and those that have been blocked.
6. Washing the particles over a magnet with an alkaline buffer to remove substrates from the previous reactions, dehybridize the miRNAs (keeping the cDNA that is already bound to the particle) and remove molecules that are not bound to the particle. Then, equilibrate the pH with another buffer. Of note, equilibrating the pH means returning to a pH at which the DNA (or RNA) strands can hybridize with high efficiency and the molecule has maximum stability. The optimum pH for nucleic acids is around 7.8, although in this example the inventors use the physiological pH (7.4 in blood).

The goal of this step it is to denature (dehybridize) the nucleic acid double helix and to sediment the particles under denaturing conditions. In this case, particles are sedimented with a magnet because they are magnetic particles, but other particles may be sedimented by centrifugation. Once sedimented, the supernatant can be easily removed, thereby washing out any molecule (DNA or RNA) that is not covalently bound to the particle. There are several ways to denature DNA (or RNA), of which the most common are:
- Increasing the temperature: the temperature of denaturation (also referred to as the melting temperature) depends on the composition of the DNA (or RNA). A temperature of 95 °C ensures the denaturation of all DNA (or RNA) molecules.
- Using an alkaline buffer: above pH 10 the nitrogenous bases of the DNA (or RNA) are ionized. This creates an electrostatic repulsion between the two strands, which ends up breaking the H-bridges that keep them in hybridization.

In the present example the inventors use an alkaline 200 mM NaOH solution to denature the DNA. It is also possible to use a buffer (e.g., carbonate) at pH 10-11. Sedimentation must be performed under denaturing conditions. In general, it is much simpler and more convenient to sediment particles at high pH rather than at a high temperature.

Other methods for denaturing DNA (or RNA) have been described, including the use of organic compounds such as formaldehyde or formamide, which intercalate the double helix and break the H-bridges.

7. Adding a poly(G) tail to the 3' end of the cDNA strand **(****Figure 4E****).** The tailing can also be performed with another nucleotide, but the use of guanines is preferred because: i) the enzyme terminal deoxynucleotidyl transferase (TdT), which is the enzyme used in this case, is more efficient incorporating dGTP than any other dNTP, and ii) the G-C bond is stronger than the A-T bond. This favors a more efficient hybridization with the second oligonucleotide.

8. Washing as indicated in step 6.

9. Using the previously added poly(G) tail as a template to hybridize a synthetic oligonucleotide that comprises the sequence of a second adapter and a barcode for indexing the samples, and elongating said synthetic oligonucleotide **(****Figure 4F****).** The presence of a barcode in the second oligonucleotide is optional. Once again, it is important to note that the adapter is not added through a ligation reaction, thereby eliminating the ligation bias associated to other methods used for the generation of cDNA libraries.

10. Washing as indicated in step 6.

11. Performing a standard PCR using the adapters as the template for the oligonucleotides and thereby remove the particles of the molecules of interest, which form the miRNA population library **(****Figure 4G****).**

The method herein described was successfully used to generate complete libraries derived from synthetic miRNA with a very high sensitivity, as described in **Example 2.3.**

The methodology of the invention can be used:
- for the generation of cDNA libraries derived from RNA samples, especially from small RNA samples (including miRNA). This method enables the identification of new and unknown variants of RNAs.
- for the non-specific amplification of small RNA molecules (including miRNA) present in a sample. For this purpose, it is sufficient to change the sequences of the adapters, which would have the sole purpose of binding primers for PCR amplification in the final stage. Non-specific amplification can be associated with microarray identification (and quantification) techniques. Microarrays identify a battery of specific miRNAs by hybridization with probes attached to a substrate. It is a relatively inexpensive method but is limited by sensitivity. The detection threshold of microarrays is around 50 nM, which limits the applicability of the technology in clinical practice, since the concentration of multiple miRNAs in blood is below 50 nM. The present invention can be used to increase the sensitivity of microarrays, preferably microarrays based on the detection of a bioluminescent signal by using a photomultiplier, which have been successfully used to identify and quantify dozens of miRNAs in the same sample. The combination of microarrays with the present invention could bring the sensitivity of the microarrays below to 0.01 nM (3 orders of magnitude). This improvement in sensitivity is key, since it would enable the identification and quantification of clinically relevant miRNAs that are present at a low concentration in biological samples.
- This can in turn be used:
   ∘ to increase the sensitivity of techniques based on the hybridization with probes on a surface (e.g., RNA microarrays).
   ∘ to determine the level of a small RNA present in sample obtained from a subject, for instance, for diagnostic purposes.

Although the use of CC reactions is not essential, their incorporation into the methodology increases efficiency in the first steps and greatly reduces preparation times:
- In terms of efficiency, the inventors were able to capture miRNAs in solution at a 100-fold lower concentration compared to previous methodologies in which steps 2 and 3 were performed on the surface of a particle.
- In terms of time, the capture of miRNAs in the present procedure takes 1.5 hours, whereas in previous methodologies this step required an overnight incubation.

The first embodiment of the invention refers to a method for the generation of a cDNA library from a RNA sample, which comprises:
a) providing an adenylated RNA,
b) hybridizing the adenylated RNA with a first synthetic oligonucleotide that comprises a poly T tail,
c) reverse-transcribing the hybridized RNA to cDNA,
d) attaching the cDNA strand to a functionalized particle through a chemical bond,
e) de-hybridizing the RNA to expose the cDNA,
wherein the steps (b) and/or (c) are performed in solution, and in that method does not comprise ligation reactions between oligonucleotides.

In a preferred embodiment the method is for the generation of a cDNA library from an RNA sample for the non-specific amplification of small RNA molecules.

In a preferred embodiment, the method optionally comprises:
i) adding an oligonucleotide tail to the cDNA molecule after step (e), and
ii) optionally, hybridizing the cDNA of (i) with a second synthetic oligonucleotide that comprises a sequence complementary to the oligonucleotide tail, and
iii) optionally using the synthetic oligonucleotide of (ii) as a primer for the synthesis of the complimentary DNA strand,
wherein in step (d) the cDNA is attached to the functionalized particle through a chemical bond between a chemical group present in the functionalized particle and a chemical group present in the first or second synthetic oligonucleotide.

In a preferred embodiment, the steps are performed in the following order: (a), (b), (c), (d), (e); or (a), (b), (c), (e), (d).

In a preferred embodiment, step b) further comprises eliminating, inactivating, or preventing the elongation of the unhybridized synthetic oligonucleotide.

In a preferred embodiment, the elimination, inactivation, or prevention of the elongation of the unhybridized synthetic oligonucleotide is performed using a method selected from: blocking the 3' end of the oligonucleotide using ddTTP, eliminating biotinylated oligonucleotides using streptavidin-associated particles, introducing a sequence in the oligonucleotide designed to hybridize 3' end of the oligonucleotide in a hairpin structure.

In a preferred embodiment, the chemical bond is a covalent bond, preferably a triazole bond.

In a preferred embodiment, the chemical group present in the functionalized particle is selected from: an azide group, an alkyne group, a carboxyl group, an amino group, a phenyl azide group and a cyclooctyne group.

In a preferred embodiment, the chemical group present in the first or second synthetic oligonucleotide is a chemical group present at the 5' end of the first synthetic oligonucleotide.

In a preferred embodiment, step a) is performed using an enzyme selected from: *Escherichia coli* poly(A) polymerase and yeast poly(A) polymerase.

In a preferred embodiment, the addition of the oligonucleotide tail to the DNA molecule after step d) or e) is performed using the enzyme terminal deoxynucleotidyl transferase (TdT).

In a preferred embodiment, the linker is selected from: propargylamine and poly-ethylene glycol (PEG) linkers.

In a preferred embodiment the oligonucleotide tail is a poly G tail.

In a preferred embodiment the RNA is a small RNA, preferably a small RNA selected from: microRNA (miRNA), Piwi-interacting RNA (piRNA), small interfering RNA (siRNA), small nuclear RNA (snRNA), also commonly referred to as U-RNA, small nucleolar RNA (snoRNA), small rDNA-derived RNA (srRNA), tRNA fragment (tRF) and Y RNA-derived small RNA (ysRNA).

In a preferred embodiment, if an enzyme has been used to carry out a given step, an inactivation of the enzyme is performed prior to starting the next step.

The second embodiment of the present invention refers to an *in vitro* method for detecting, identifying and/or determining the level of an RNA present in sample obtained from a subject, the method comprising generating cDNA from RNA isolated from a sample by following the method of any of the claims 1 to 8 and amplifying or quantifying the cDNA or a complementary strand thereof by using preferably PCR.

In a preferred embodiment, the *in vitro* method for detecting, identifying and/or determining the level of an RNA present in a sample further comprises detecting the RNA using a microarray.

The third embodiment of the invention refers to a method for the generation of a functionalized particle comprising an alkyne group or an azide group, or for the modification of particles comprising a carboxyl group into particles comprising an alkyne group or an azide group, which comprises:
a) Providing particles comprising a carboxyl group,
b) Incubating the particle of step (a) in the presence of a linker comprising an amine group in one end, and an alkyne group or an azide group in the other end.

In a preferred embodiment, the functionalized particle is a magnetic particle.

The fourth embodiment of the invention refers to a functionalized particle obtained by the method of the invention for the generation of a functionalized particle.

The fifth embodiment of the invention refers to a particle functionalized with an azide or an alkyne chemical group.

In a preferred embodiment, the functionalized particle or particle is a magnetic particle.

The fifth embodiment of the invention refers to the use of any of the particles of the invention for carrying out any of the methods of the invention.

In a preferred embodiment, the particle or functionalized particle is a micrometric or nanometric nanoparticle.

### In the context of the present invention the following terms are defined:

- The term "comprising" means including, but it is not limited to, whatever follows the word "comprising". Thus, use of the term "comprising" indicates that the listed elements are required or mandatory, but that other elements are optional and may or may not be present.
- The term "consisting of" means including, and it is limited to, whatever follows the phrase "consisting of'. Thus, the phrase "consisting of" indicates that the listed elements are required or mandatory, and that no other elements may be present.
- The expression "attaching the cDNA strand to a functionalized particle through a chemical bond" refers to attaching the cDNA strand trough a particle through a chemical bond between a chemical group that is somehow attached to the nanoparticle and a chemical group that is somehow attached to the cDNA. The chemical group attached to the cDNA is not necessarily present in the cDNA strand. Instead, it may be present in an oligonucleotide that is hybridized with the cDNA through base pair complementarity.
- The expression "de-hybridizing the RNA to expose the cDNA" refers to performing a reaction that results in the exposure of the de-hybridized cDNA strand, so that it can interact by sequence complementary with other oligonucleotides. A person skilled in the art would be able to recognize the methods through which this can be achieved, some of which involve the degradation of the RNA molecule. For instance, it may be achieved using a ribonuclease enzyme, a type of nuclease that catalyzes the degradation of RNA - but not the cDNA - into smaller components.
- The expression "in solution" means that the molecules involved in the step that is performed in solution are not attached to a surface, let alone to the surface of a particle.
- The term "providing an adenylated RNA" refers to the fact that is necessary to depart from a population of RNA that is polyadenylated in the 3' end of the molecule. The 3'-ends of both prokaryotic and eukaryotic messenger RNA (mRNA) are polyadenylated, meaning that these molecules do not have to be polyadenylated prior to starting the claimed method for the generation of a cDNA library. Other RNA molecules, such as miRNAs, are not naturally polyadenylated and therefore a poly(A) tail must be added to their 3' end for them to the prepared for the method of the invention. Polyadenylation may be performed using commercially available enzymes and protocols. It is a well-established procedure in molecular biology.
- The term "RNA sample" refers to a sample that comprises any type of RNA molecule, including small RNA (sRNA) molecules. sRNA are polymeric RNA molecules that are less than 200 nucleotides in length and are usually non-coding. Types of small RNA include: microRNA (miRNA), Piwi-interacting RNA (piRNA), small interfering RNA (siRNA), small nuclear RNA (snRNA), also commonly referred to as U-RNA, small nucleolar RNA (snoRNA), small rDNA-derived RNA (srRNA), tRNA fragment (tRF) and Y RNA-derived small RNA (ysRNA).

### Description of the figures

**Figure 1****.** Scheme of the Click Chemistry (CC) triazole bond.
**Figure 2****.** Schematic representation of the amide reaction between the magnetic particle and propargylamine.

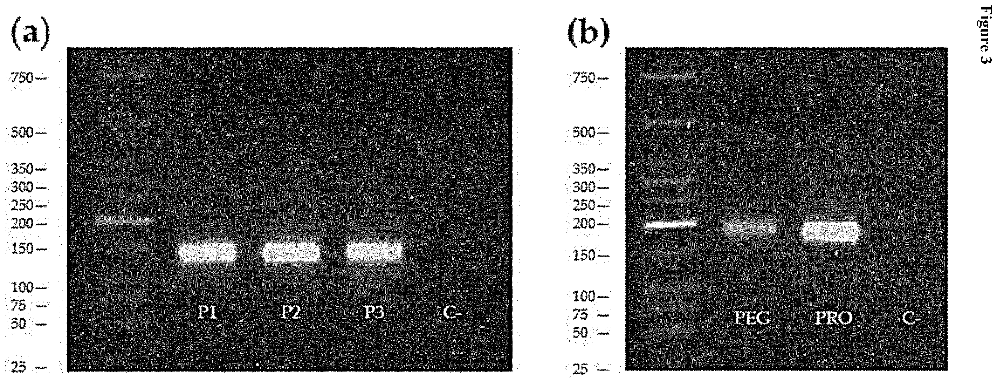

**Figure 3****.** Electrophoresis gels. **(A)** DNA result of functionalized particles that were functionalized 6 weeks prior to the experiment (P1), particles were functionalized 5 weeks prior(P2) and particles were functionalized 3 weeks prior (P3). **(B)** In P1, amino-PEG4-alkyne particles were used, whereas in P2, propargylamine particles were used. C- are PCR controls.
**Figure 4****.** General Scheme of the Methodology. **(A)** Polyadenilation of miRNAs by poly(A) polymerase. **(B)** miRNAs are hybridized to 5' azide-modified oligonucleotides and then retrotranscribed. **(C)** 5' azide-modified oligonucleotides that had not hybridize a miRNA are blocked. **(D)** A click chemistry reaction is done to bind miRNAs to magnetic functionalized particles. **(E)** A guanidine's tail is added by TdT enzyme. **(F)** One adapter is elongated by GoTaq. **(G)** Finals PCRs are done to add the rest of adapters and the index and to release the DNA from the particle. This imagen is not to scale. The sequences included in this Figure are: SEQ ID NO: 1 AAAAAAAAAAAAAAAAAAAAAAA **(****Fig. 4A****),** SEQ ID NO: 2 AAAAAAAAAAAAAAAAAAAAA **(****Fig. 4B****).** SEQ ID NO: 3 TTTTTTTTTTTTTTTTTTTTT **(****Fig. 4B****),** SEQ ID NO: 4 AAAAAAAAAAAAAAAAAAAAA **(****Fig. 4B****),** SEQ ID NO: 5 TTTTTTTTTTTTTTTTTTTTT **(****Fig. 4B****),** SEQ ID NO: 6 AAAAAAAAAAAAAAAAAAAAAAAAAA **(****Fig. 4C****),** SEQ ID NO: 7 TTTTTTTTTTTTTTTTTTTT **(****Fig. 4C****),** SEQ ID NO: 8 TTTTTTTTTTTTTTTTTTTT **(****Fig. 4C****),** SEQ ID NO: 9 TTTTTTTTTTTTTTTTTTTT **(****Fig. 4D****),** SEQ ID NO: 10 TTTTTTTTTTTTTTTTTTTT **(****Fig. 4D****),** SEQ ID NO: 11 TTTTTTTTTTTTTTTTTTTT **(****Fig. 4E****),** SEQ ID NO: 12 GGGGGG **(****Fig. 4E****),** SEQ ID NO: 13 TTTTTTTTTTTTTTTTTT **(****Fig. 4E****),** SEQ ID NO: 14 TTTTTTTTTTTTTTTTTT **(****Fig. 4E****),** SEQ ID NO: 15 TTTTTTTTTTTTTTTTTT **(****Fig. 4E****),** SEQ ID NO: 16 TTTTTTTTTTTTTTTTTT **(****Fig. 4E****),** SEQ ID NO: 17 CCCCCC **(****Fig. 4G****),** and SEQ ID NO: 18 GGGGGG **(****Fig. 4G****).**

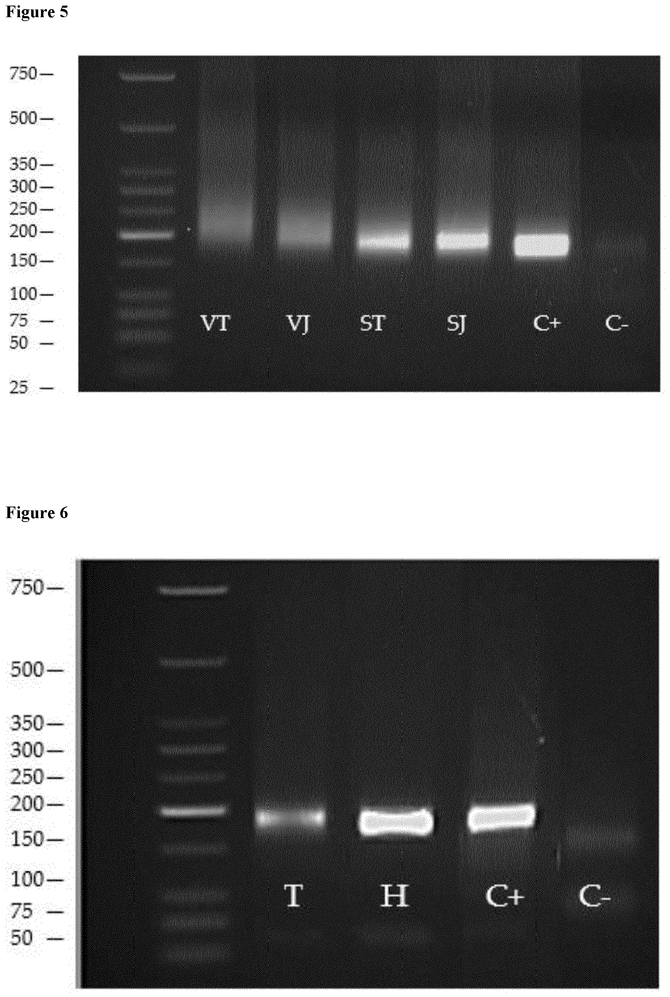

**Figure 5****.** Electrophoresis gel. Comparison of Yeast Poly(A) Polymerase from Thermo Fisher Scientific (VT and ST) and Yeast Poly(A) Polymerase from Jena Bioscience (VJ and SJ). DNA result of miRNA libraries of adipose tissue in VT and VJ and Standards libraries in in ST and SJ where 20 pM Standards were adenylated. C+ is a positive control with a concentration of 40 pM of Simulator. C- is a PCR control.
**Figure 6****.** Electrophoresis gel. In T, blocking A has been performed. In H, blocking B has been per-formed. C+ is a positive control with a concentration of 40 pM of Simulator. C- is a PCR negative control.
**Figure 7****.** Electrophoresis gel. Standards were used for this experiment. P1: 0.2 nM of Standards and 10 nM of 5' azide-modified oligonucleotide, P2: 0.2 nM of Standards and 6 nM of 5' azide-modified oligonucleotide, P3: 20 pM of Standards and 6nM of 5' azide-modified oligonucleotide, P4 and P5: 2 pM of Standards and 6nM of 5' azide-modified oligonucleotide. In P4, click chemistry incubation lasted for 1h, in the rest click chemistry reaction was incubated overnight. C+ is a positive control with a concentration of 40 pM Simulator. C- is a PCR negative control. In this occasion we can see two bands in some samples as there were oligonucleotides which have not been properly blocked.
**Figure 8****.** Electrophoresis gels of PCR controls. **(A)** C1 is a PCR negative control when performing just one final PCR with 40 cycles. **(B)** C2 is a PCR A negative control with 20 cycles. C3 is a PCRA+B negative control with 20 cycles of PCR A plus 40 cycles of PCR B. C4 is a PCR B negative control with 40 cycles.

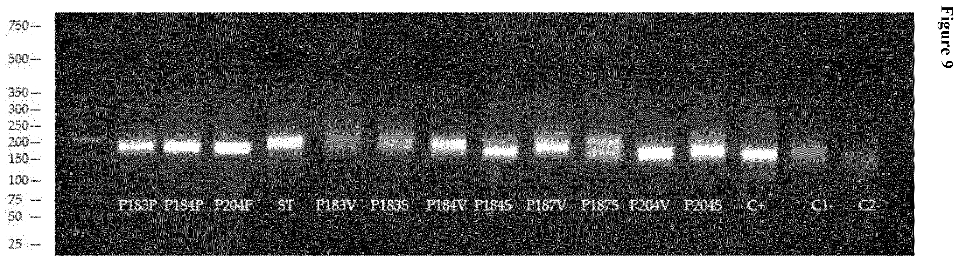

**Figure 9****.** Final libraries in electrophoresis gel. In P183P, P184P and P204P plasma samples were used. In ST, 0.4 nM Standards were used. In P183V, P184V, P187V and P204V VAT samples were used. In In P183S, P184S, P187S and P204S SAT samples were used. C+ is a positive control where 40 pM Simulator was used (positive control). C1- and C2- are PCR negative controls.

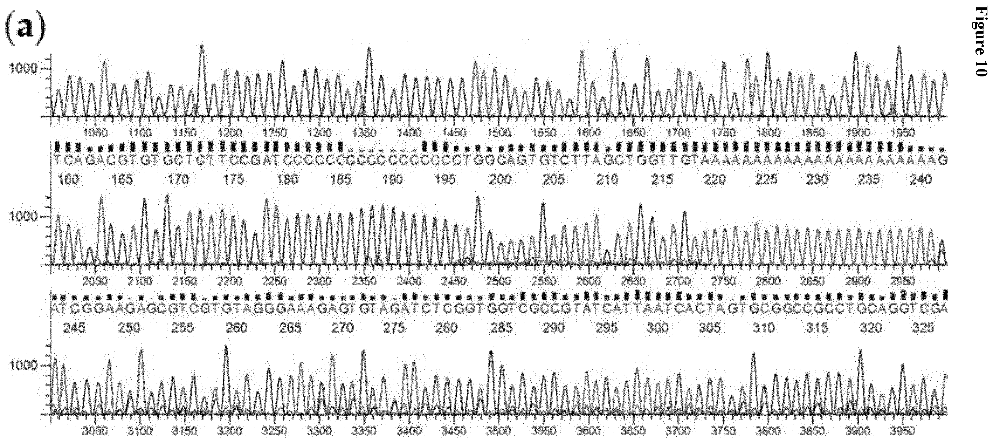

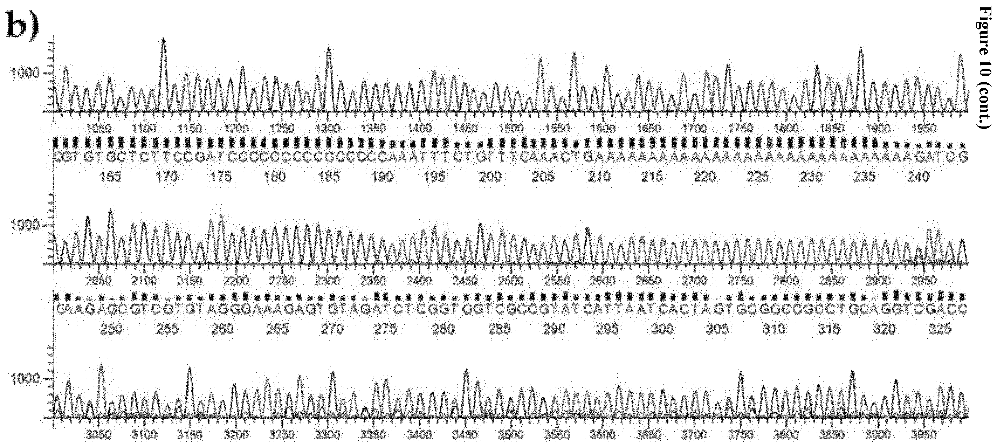

**Figure 10****.** Standards library sanger sequencing. **(A)** The sequences of adapters, including G's and A's tails (represented here as C's and T's), along with the Standard 4 sequence, are presented. **(B)** In the case of the VAT library prepared for Sanger sequencing, the adapters' sequences, encompassing G's and A's tails (shown as C's and T's), as well as a potential
miRNA sequence, are illustrated. The sequences included in this Figure are: SEQ ID NO: 19 TCAGACGTGTGCTCTTCCGATCCCCCCCCCCCCCCCCCTGGCAGTGTCTTAGCTGG TTGTAAAAAAAAAAAAAAAAAAAAAAAGATCGGAAGAGCGTCGTGTAGGGAAA GAGTGTAGATCTCGGTGGTCGCCGTATCATTAATCACTAGTGCGGCCGCCTGCAG GTCGA **(****Fig. 10A****),** SEQ ID NO: 20: CGTGTGCTCTTCCGATCCCCCCCCCCCCCCCAAATTTCTGTTTCAAACTGAAAAA AAAAAAAAAAAAAAAAAAAAAAAAAGATCGGAAGAGCGTCGTGTAGGGAAAG AGTGTAGATCTCGGTGGTCGCCGTATCATTAATCACTAGTGCGGCCGCCTGCAGG TCGACC **(****Fig. 10B****).**

### Detailed description of the invention

The present invention is illustrated by means of the Examples set below without the intention of limiting its scope of protection.

### Example 1. Material and Methods

### Example 1.1. Study Samples

Blood and adipose tissue (AT) samples were collected from patients who underwent bariatric surgery. The hospital ethics committee approved this study, and all participants signed a written since this master's thesis is included in a previous existing project and doctoral thesis. Patients were excluded from the cohort if they presented any autoimmune disorder.

### Example 1.2. Synthetic miRNA

30 oligonucleotides that simulate existing miRNA (referred to as Standards) were purchased from IDT (Integrated DNA Technologies, Inc., Coralville, IA, USA) as well as 1 oligonucleotide that simulate a miRNA that included a polyadenyl tail (referred to as Simulator hereafter) **(Table 1).** The Silmulator was use during the development of the methodology in order to avoid the adenylation step.

### Example 1.3. Plasma miRNA Isolation

Isolation of miRNA was performed with miRNeasy Serum/Plasma Advanced Kit (QIAGEN Gmbh, Hilden, Germany) according to the manufacturer's guidelines. Frozen plasma samples were placed at room temperature and miRNA was isolated from 200 µl of plasma. Final volume of miRNA isolation was 20 µL and was stored at -20°C prior to miRNA library preparation. The concentration of miRNA was measured using a Qubit miRNA Assay (Thermo Fisher Scientific Inc., Waltham, MA, USA) and a Qubit 3.0 fluorimeter (Thermo Fisher Scientific Inc., Waltham, MA, USA).

### Example 1.4 Tissue miRNA Isolation

Two types of adipose tissue were obtained per patient, subcutaneous adipose tissue (SAT) and visceral adipose tissue (VAT). Approximately 200 mg of tissue per sample was processed with a manual homogenizer and the isolation of miRNA was performed following the manufacturer's instructions of ReliaPrep miRNA Cell and Tissue Miniprep System (Promega, Madison, WI, USA). Final volume of tissue miRNA isolation was 40 µL and was stored at -20°C prior to miRNA library preparation. The concentration of miRNA was measured using a Qubit miRNA Assay (Thermo Fisher Scientific Inc., Waltham, MA, USA) and a Qubit 3.0 fluorimeter (Thermo Fisher Scientific Inc., Waltham, MA, USA).

### Example 1.5. Magnetic particles functionalization

Three different magnetic particles were used in this study as described in Results: Sera-Mag^{™}SpeedBead E3, Sera-Mag^{™}SpeedBead E7 (Cytiva, Marlborough, MA, USA) and Dynabeads^{™}MyOne^{™}Carboxylic Acid (Thermo Fisher Scientific Inc., Waltham, MA, USA). All of them have a size of approximately 1 µm. They were functionalized with amino-PEG4-alkyne and propargylamine from Sigma-Aldrich (Merck KGaA, Darmstadt, Germany) using *N*-etil-*N'*-(3-dimetilaminopropil) carbodiimide hydrochlo-ride (EDAC) from Sigma-Aldrich (Merck KGaA, Darmstadt, Germany).

### Example 1.6. Library preparation

The libraries were prepared in accordance with the procedures outlined in the Results section, employing the specified materials mentioned for the execution of each step.

### Example 1.7. Electrophoresis

A 2.2% agarose gel was prepared where 15 µl of PCR product was loaded and 6 µl of Low Molecular Weight DNA Ladder (New England Biolabs Inc., Ipswich, MA, USA).

### Example 1.8. Library purification

The final PCR B products were purified using ProNex Size-selective Purification System (Promega, Madison, WI, USA) according to the manufacturer's guidelines, adding 2.2 times the volume of PRC product in particles.

### Example 1.9. Bacterial cloning and transformation

For the ligation reaction, 7 µl of library purification was used with 1 µl of vector pGEM-T (Promega, Madison, WI, USA), 2 µl ligase and 10 µl reaction buffer. Ligation reaction was held at room temperature for several hours (minimum of 1 hour). For the bacterial transformation, 50 µl JM109 Competent Cells (Promega, Madison, WI, USA) were gently mixed with the ligation, and then placed on ice for 30 minutes. They were given a thermal shock at 42 degrees for 45 seconds and then incubated for 5 minutes on ice. Afterwards, 500 µl LB Broth liquid medium (Condalab, Madrid, Spain) were added and incubated in a shaker at 37 °C for 1 hour. After transformation, competent cells were inoculated in freshly prepared 30 ml LB agar plates containing 80 mg/ml of X-Gal (Promega, Madisgon, WI, USA), 30 µl of IPTG 1000x and 30 µl of ampicillin 100 mg/ml. JM109 were let to grow overnight and then placed at 5 °C for 10 minutes to better discriminate in between positive and negative colonies. Several colonies of bacteria were selected and grown in LB broth with ampicillin overnight under the condition of shaking at 37 °C.

### Example 1.10. Sanger sequencing

After growth overnight, plasmids from bacteria were obtained using wizard Plus SV minipreps DNA purification system (Promega, Madison, WI, USA) according to the manufacturer's guidelines. The concentration of DNA was measured using Qubit miRNA Assay (Thermo Fisher Scientific Inc., Waltham, MA, USA) and a Qubit 3.0 fluorimeter (Thermo Fisher Scientific Inc., Waltham, MA, USA).

Samples were sent to the Genomics Unit of the Institute of Parasitology and Bio-medicine Lopez-Neyra to proceed with Sanger sequencing. DNA sequencing was performed by the cycle sequencing procedure using BigDyeTerminator v3.1 chemistry and automated multicapillary system electrophoresis. The primer used for Sanger sequencing is located on the pGEM-T vector, right before the start of our sequence of interest so that the background noise at the beginning of the sequence is avoided.

### Example 2. Results

### Example 2.1. Magnetic particles functionalization

The inventors used three different magnetic particles to test their efficiency: Sera-Mag^{™}SpeedBead E3, Sera-Mag^{™}SpeedBead E7 and Dynabeads^{™}MyOne^{™}Carboxylic Acid. They are covered with carboxylic groups on their surface that are used to couple different molecules with amine ends, creating an amide covalent bond. This chemical reaction relies on the carbodiimide reactant to create an intermediate group referred to as active carboxyl. The molecules coupled to the beads have an alkyne end at the other termination, which will be used to perform a click chemistry reaction. This reaction will covalently couple any oligonucleotide with an azide end.

Beads were used at a final concentration of 12.5 mg/ml and *N*-etil-*N'*-(3-dimetilaminopropil) carbodiimide hydrochloride (EDAC) was used at a concentration of 0.24 mg/µl. Different batches of particles were functionalized with two different molecules, amino-PEG4-alkyne and propargylamine from Sigma-Aldrich^{®}. The concentration of these molecules varies depending on the experiment. The reaction is carried out at acidic pH (between 4.8 and 6) using MES buffer.

Firstly, the particles were washed with MES buffer several times using a magnet. EDAC and amino-PEG4-alkyne or propargylamine were added to the particles resuspended in MES buffer and shaken vigorously for 10 minutes. Then, beads were incubated for 4 hours in constant slow rotation. Afterwards, washes with sodium hydroxide (NaOH), Tris-HCl 200 mM and Tris-HCl 50 mM were performed to stop the reaction and restore the pH level. The modified magnetic particles were used right after or were stored at 5°C prior to their use.

Stability tests were done using extreme pHs and temperature. Sera-Mag^{™}Speed-Bead E7 and Dynabeads^{™}MyOne^{™}Carboxylic Acid were unable to pass the tests. The amount of particles decreased after being washed with NaOH. They sedimented rapidly even without the need for a magnet (indicating aggregation among themselves, making certain areas of the particles inaccessible to enzymes and masking part of the surface). Additionally, they irreversibly adhered to the walls of tubes, including low binding and siliconized ones, resulting in sample loss. Hence, Sera-Mag^{™}SpeedBead E3 showed the most stability throughout all the process. Moreover, they were stable over time **(****Figure 3A****),** so it was not necessary to prepare fresh particles for every experiment. On the other hand, particles were functionalized with two different molecules, amino-PEG4-alkyne and propargylamine. Even though both worked, those functionalized with propargylamine obtained better results. As seen in **Figure 3B****,** when using propargylamine-coated particles (PRO band in **Figure 3B****),** a higher amount of DNA is obtained.

### Example 2.2. Library preparation

In the following section, the steps to prepare high-throughput sequencing libraries using the proposed methodology are described **(****Figure 4****).** To begin with, it must be noted that in all preclick chemistry reactions MMLV buffer (Promega, Madison, WI, USA) was used with not significant differences with those where each enzyme-specific buffer was used. Likewise, a "Simulator", a synthetic miRNA with a poly-A tail added, was used as a positive control in these experiments **(Table 1).**

**Table 1. Simulator and Standards.**

| **Name** | **Sequence** | **SEQ ID** |
|---|---|---|
| Simulator | AGA AGC AGA GAU CUC ACA CAA AAA AAA AAA AAA AAA AAA AA | SEQ ID NO: 21 |
| P1-miR-135a | UAU GGC UUU UUA UUC CUA UGU GA | SEQ ID NO: 22 |
| P2-miR-135b | UAU GGC UUU UCA UUC CUA UGU GA | SEQ ID NO: 23 |
| P3-miR-26b | UUC AAG UAA UUC AGG AUA GGU | SEQ ID NO: 24 |
| P4-miR-34a | UGG CAG UGU CUU AGC UGG UUG U | SEQ ID NO: 25 |
| P5-miR-34b | UAG GCA GUG UCA UUA GCU GAU UG | SEQ ID NO: 26 |
| P6-miR-34c | AGG CAG UGU AGU UAG CUG AUU GC | SEQ ID NO: 27 |
| P7-miR-145 | GUC CAG UUU UCC CAG GAA UCC CU | SEQ ID NO: 28 |
| P8-miR-125b | UCC CUG AGA CCC UAA CUU GUG AAA CTT GTGA | SEQ ID NO: 29 |
| P9-miR-320a | AAA AGC UGG GUU GAG AGG GCG A | SEQ ID NO: 30 |
| P10-miR-23b | AUC ACA UUG CCA GGG AUU ACC AC | SEQ ID NO: 31 |
| P11-miR-17 | CAA AGU GCU UAC AGU GCA GGU AG | SEQ ID NO: 32 |
| P 12-miR-18a | UAA GGU GCA UCU AGU GCA GAU AG | SEQ ID NO: 33 |
| P13-miR-20a | UAA AGU GCU UAU AGU GCA GGU AG | SEQ ID NO: 34 |
| P14-miR-21 | UAG CUU AUC AGA CUG AUG UUG A | SEQ ID NO: 35 |
| P15-miR-23a | AUC ACA UUG CCA GGG AUU UCC | SEQ ID NO: 36 |
| P16-miR-24 | UGG CUC AGU UCA GCA GGA ACA G | SEQ ID NO: 37 |
| P17-miR-29c | UAG CAC CAU UUG AAA UCG GUU A | SEQ ID NO: 38 |
| P18-miR-125a | UCC CUG AGA CCC UUU AAC CUG UGA | SEQ ID NO: 39 |
| P19-miR-130a | CAG UGC AAU GUU AAA AGG GCA U | SEQ ID NO: 40 |
| P20-miR-150 | UCU CCC AAC CCU UGU ACC AGU G | SEQ ID NO: 41 |
| P21-miR-200c | UAA UAC UGC CGG GUA AUG AUG GA | SEQ ID NO: 42 |
| P22-miR-210 | CUG UGC GUG UGA CAG CGG CUG A | SEQ ID NO: 43 |
| P23-miR-221 | AGC UAC AUU GUC UGC UGG GUU UC | SEQ ID NO: 44 |
| P24-miR-301a | CAG UGC AAU AGU AUU GUC AAA GC | SEQ ID NO: 45 |
| P25-miR-365a | UAA UGC CCC UAA AAA UCC UUA U | SEQ ID NO: 46 |
| P26-miR-454 | UAG UGC AAU AUU GCU UAU AGG GU | SEQ ID NO: 47 |
| P27-miR-663b | GGU GGC CCG GCC GUG CCU GAG G | SEQ ID NO: 48 |
| P28-miR-synthetic1 | GAA GUC CUC UUC CAA CUC CUG UUG | SEQ ID NO: 49 |
| P29-miR-synthetic2 | GUA GGU UCA CUC CUG UGU UCC UAC | SEQ ID NO: 50 |
| P30-miR-synthetic3 | UGG AUC ACC AUG GAC UAC CA | SEQ ID NO: 51 |

A poly-A tail was added to the miRNA samples and to the Standards (synthetic miRNAs) **(Table 1).** Two different Yeast Poly(A) Polymerase were tested to add this tail, Yeast Poly(A) Polymerase (Jena Bioscience GmbH, Jena, Germany) and Poly(A) Polymerase (Thermo Fisher Scientific Inc., Waltham, MA). The polyadenylation was carried out according to the manufacturer's protocol incubating the reaction for 30 minutes at 37°C, adding 5 µl MMI,V buffer 5x, 2.5 µl ATP at a concentration of 2 mM, 0.25 µl of enzyme and 17.25 µl of miRNA sample or Standards (concentration may vary depending on the experiment). It was stopped by adding EDTA at a final concentration of 3.7 nM.

Despite both being able to add the adenyl tail, Yeast Poly(A) Polymerase from Jena Bioscience was the enzyme which retrieve better results as seen in **Figure 5****.** The adenyl tail length should be greater than 20 nucleotides because of the length of the T tail at the 3' end of the 5' azide-modified oligonucleotide. The goal is to ensure that the 3' end of the polyT tail is always hybridized and can elongate. For instance, the poly-A tail should be 25-30 nucleotides. Both enzymes added a variable number of adenines as seen in **Table 2.** Because of this, it's difficult to obtain a define band in the electrophoresis gel. Nevertheless, when using Yeast Poly(A) Polymerase from Jena Bioscience, there is a higher amount of DNA and a more define band (VJ and SJ bands versus VT and ST bands in **Figure 5****,** respectively).

**Table 2. Poly-A polymerases' performance.**

| **Enzyme** | **A's added (mean ± standard deviation)** |
|---|---|
| Yeast Poly(A) Polymerase by Thermo Fisher | 24.5 ± 6.5 |
| Yeast Poly(A) Polymerase by Jena Bioscience | 26.7±1.82 |

### Reverse transcription of miRNAs and hybridization with oligonucleotides

To retrotranscribe the miRNA to cDNA and to add the azide end needed to proceed with click chemistry, the 5' azide-modified oligonucleotide **(Table 3)** and the poly-adenylated miRNA were hybridized. The 5' azide-modified oligonucleotide consists of a part of the sequence of one of the P5-SP1 adapters for NGS and a poly-T tail at its 3' end that will hybridize with the miRNA population. All the reagents were added in a total volume of 55 µl, minus the MMLV retrotranscriptase enzyme. The reaction volume consisted of polyadenylation product, 7 µl of 5X MMLV buffer, 5.5 µl of dNTPs at a concentration of 2 mM, and 6 µl of MgCl2. The azide-modified oligonucleotide (refer to **Table 3)** was employed at varying concentrations based on the specific experiment, but finally it was standardized to a concentration of 6 nM. In instances where EDTA was not a concern from the preceding step, such as in Simulator, H2O was added instead of MgCl2. To perform the hybridization, the incubation was as follows: 95 °C for 3 min, 70 °C for 5 min, 65 °C for 5 min, 60 °C for 5 min, 55 °C for 5 min, 50 °C for 5 min. When the incubation was finished, the retrotrascriptase enzyme was added, and the RT reaction was incubated for 1 hour at 37 °C.

**Table 3. Primers and molecules used in the different steps of the experiments as described before.**

| **Reaction** | **Name** | **Sequence (5' - 33)** | **SEQ ID** |
|---|---|---|---|
| miRNA hybridization | 5' azide-modified oligonucleotide | AC ACT CTT TCC CTA CAC GAC GCT CTT CCG ATC TTT TTT TTT TTT TTT TTT TT The sequence comprises an azide group in the 5' end. | SEQ ID NO: 52 |
| Blocking A | Bead-Inactivation | AAA AAA AAA AAA AAA AAA AAA AAA AAA GAT CGG AAG AGC GTC GTG TAG G | SEQ ID NO: 53 |
| Blocking B | Hairpin | GCG CCA CGA TTC AAC GTG GCG CAA AAA AAA AAA AAA AAA AAA GAT CGG AAG AGC GTC GTG TA | SEQ ID NO: 54 |
| Adapters elongation | Ant-miRNA-elongation | GTG ACT GGA GTT CAG ACG TGT GCT CTT CCG ATC CCC CCC CCC CCC CCC CCC C | SEQ ID NO: 55 |
| Final PCR | Sen- Fin-A | CAC CGA GAT CTA CAC TCT TTC CCT ACA CGA | SEQ ID NO: 56 |
| | Sen- Fin-B | AAT GAT ACG GCG ACC ACC GAG ATC TAC A | SEQ ID NO: 57 |
| | Ant- Fin-A-X | The sequence of the index (which must vary for each sample) flanked by CGG CAT ACG AGA T (SEQ ID NO: 58) in the 5' end and T GAC TGG AGT TCA GAC G (SEQ ID NO: 59) in the 3' end. | SEQ ID NO: 58 SEQ ID NO: 59 |
| | Ant- Fin-B: | 5'-CAA GCA GAA GAC GGC ATA CGA G-3' | SEQ ID NO: 60 |

### Oligonucleotide blocking

5' azide-modified oligonucleotides that have not hybridized with polyadenylated miRNAs (and elongated with the sequence of miRNAs during RT) must be blocked so they will not continue with the following steps. This procedure becomes more relevant when we have very limited quantities of small RNA.

Two types of blocking were used: introducing a dideoxynucleotide, specifically a ddTTP, by a Therminator DNA Polymerase (New England Biolabs Inc., Ipswich, MA, USA) or hybridizing a hairpin-like oligonucleotide.

*Blocking A:* To add the ddTTP, the thermal cycler program used was as follows: 95 °C for 2 min, 40 cycles of 95 °C for 20 s, 60 °C for 20 s, 70 °C for 20 s. The reaction volume was 95 µl when there was a previous polyadenylation reaction and 40 µl in the Simulator case. Therminator DNA Polymerase was used according to the manufacturer's guidelines but using MMLV reaction buffer instead. ddTTPs were added at a final concentration of 4 mM and the Bead-Inactivation primer at a final concentration of 200 nM **(Table A2).**

*Blocking B:* To hybridize the hairpin, the incubation was as follows: 95 °C for 3 min, 70 °C for 5 min, 65 °C for 5 min, 60 °C for 5 min, 55 °C for 5 min, 50 °C for 5 min. A volume of 20 µl consisting of 200 nM Hairpin primer **(Table A2)** and Tris-NaCl was added to the volume of the previous reactions.

Among the two distinct blocking methods assessed, the enzymatic addition of ddTTP by Therminator (Blocking A) proved more efficiency than the Hairpin-like blocker (Blocking B) as seen in **Figure 6****.** The inventors obtained more amount of DNA when using the hairpin-like oligonucleotide (H band in **Figure 6****),** suggesting less blocking efficiency as it is comparable with the positive control. While in the Therminator result (T band in **Figure 6****),** a reduction in the amount of DNA is observed, indicating a higher oligonucleotide blocking. Of note, the efficiency of the blocking methods was assessed without adding RNA to the system. Consequently, the entire final product is derived from non-blocked oligonucleotides.

### Click chemistry

Functionalized particles (10 µl) were placed over a magnet and Tris-HCl was removed. The mixture from the previous reactions is used to resuspend the particles as well as 4 µl of CuSO4 25 mM from Sigma-Aldrich (Merck KGaA, Darmstadt, Germany) and 2 µl of sodium ascorbate from Sigma-Aldrich (Merck KGaA, Darmstadt, Germany). Sodium ascorbate is freshly prepared by adding 100 µl of water to 0.01 mg of it. For the Simulator, 1 µl of CuSO4 25 mM and 1 µl of ascorbate were used.

This reaction was carried out in a nitrogen atmosphere to avoid oxidation of the compounds by oxygen. The reaction was stopped and washed with NaOH, Tris-HCl 200 mM and Tris-HCl 50 mM except for those blocked with hairpin, where only Tris-HCl 50 mM was used. And thus, we remove from the solution all reagents that may interfere with the subsequent reactions.

Click chemistry reactions have demonstrated remarkable robustness, a quality that our study has confirmed and validated. During the development of this part of the protocol, we tested and have obtained positive results in a variety of conditions and reagents concentrations. At the beginning, we used the same concentration of azide-modified oligonucleotide and Standards. Afterwards, 10 nM of 5' azide-modified oligonucleotide was used with Standards at 0.2nM. Simultaneously, 6 nM 5' azide-modified oligonucleotide was used with Standards at 0.2 nM, 20 pM and 2 pM **(****Figure 7****).** On the other hand, different times of incubation for click chemistry were tested, between 1 hour and overnight in different experiments with no difference in performance.

### Elongation of the adapter

To add part of the other P7-SP2 adapter's sequence to the DNA in the particles, an elongation was performed. For this elongation, only one primer is needed (Ant-miRNA-elongation) **(Table A2)** and it consists of part of the sequence of SP2 and the target-binding sequence. It must be noted that this primer has a chemical modification at its 3' end that prevent it of elongation, while the DNA strands bound to the particle will elongate its 3' end with the sequence on the 5' end of the primer. The reaction mixture with GoTaq kit (Promega, Madison, WI, USA) with a final volume of 100 µl comprised: 20 µl 5x GoTaq Buffer, 6.7 µl of Ant-miRNA-elongation primer (200mM) **(Table A2),** 8 µl MgCl2 (25mM), 10 µl dNTPs (2 mM), 54.8 µl H2O and 0.5 GoTaq enzyme. The cycling conditions were as follows: pre-denaturation at 95 °C for 2 min, followed by 10 cycles at 95 °C for 20 s, 62 °C for 5 min, and 72 °C for 30 s. The particles were washed with NaOH, Tris-HCl 200 mM and Tris-HCl 50 mM, so that we will eliminate waste products and reagents from the reaction.

### Final PCRs

Two final PCRs were performed to release the DNA from the particles and complete the sequence of both adapters. PCR A was performed in a 20 µl reaction volume consisting of: 4 µl 5x GoTaq Buffer, 1.6 µl MgCl2 (25mM), 2 µl dNTPs (2 mM), 11.3 µl H2O, 0.1 GoTaq enzyme and forward and reverse primers, 0.5 µl Sen-Fin-A and 0.5 µl Ant-Fin-A-X, respectively **(Table A2).** The reverse primers were different for each sample as they must have different indexes for multiplexing in Illumina sequencer. PCR B was performed in a 50 µl reaction volume consisting of: 10 µl 5x GoTaq Buffer, 4 µl MgCl2 (25mM), 5 µl dNTPs (2 mM), 22.4 µl H2O, 0.25 GoTaq enzyme Kit, 3.33 µl forward and reverse primers, Sen-Fin-B and Ant-Fin-B, respectively **(Table A2),** and 5 µl of PCR A product (magnetic particles were carefully removed of the PCR A product using a magnet).

Final PCR had to be divided in two consecutive PCRs instead of performing one since the primers used were too long as they need to complete the sequence of the adapter. The efficiency of a unique final PCR was lower the with PCR A+B **(****Figure 8****)** and signal in the PCR negative controls was higher due to primer dimerization.

### Example 2.3. Performance of the proposed methodology

With the methodology completely developed, we validated it by constructing libraries using biological samples. Following the successful generation of libraries with minimal concentrations of standards **(****Figure 7****),** libraries were constructed using visceral adipose tissue (VAT), subcutaneous adipose tissue (SAT) and plasma samples. miRNA concentrations were quantified prior to library preparation **(Table 4).** The concentrations were higher in the tissue samples. To confirm that libraries were complete, size was checked by agarose gels **(****Figure 9****)** and sequence was checked by Sanger sequencing **(****Figure 10****).** In the initial optimization phase, we opted for *E. coli* cloning and Sanger sequencing instead of NGS, even though NGS must be done in a more advanced developmental phase. It's worth noting that cloning is a mandatory step since the library consists of a heterogeneous collection of molecules and is not suitable for Sanger sequencing.

Once the libraries were size-confirmed by the agarose gel, several samples were sequenced by Sanger to assess the methodology's efficacy and check that libraries were complete with no mistakes on them. JM109 Competent Cells were transformed with the previously made ligation in vector pGEM-T. Eight colonies were isolated coming from Standards library and eight colonies from visceral adipose tissue libraries.

Using synthetic standards (synthetic RNA of similar size and composition to miRNA) the inventors calculated a sensitivity threshold below 0.01 nM. This is two to three orders of magnitude lower than commercial library manufacturing methods and microarray detection and quantification.

**Table 4. miRNA concentration of patient samples.**

| **Patient** | **Type of sample** | **miRNA concentration (ng/pL)** |
|---|---|---|
| P183 | VAT | 71 |
| P183 | SAT | 32.2 |
| P184 | VAT | 65 |
| P184 | SAT | 30.3 |
| P187 | VAT | 50.4 |
| P187 | SAT | 11.5 |
| P204 | VAT | 72 |
| P204 | SAT | 27.1 |
| P183 | Plasma | 0.69 |
| P184 | Plasma | 0.816 |
| P204 | Plasma | 0.694 |

## Claims

1. Method for the generation of a cDNA library from an RNA sample, which comprises:
a) providing an adenylated RNA,
b) hybridizing the adenylated RNA with a first synthetic oligonucleotide that comprises a poly T tail,
c) reverse-transcribing the hybridized RNA to cDNA,
d) attaching the cDNA strand to a functionalized particle through a chemical bond,
e) de-hybridizing the RNA to expose the cDNA,
**characterized in that** the steps (b) and/or (c) are performed in solution, and **in that** the method does not comprise ligation reactions between oligonucleotides.

2. Method, according to claim 1, wherein the method optionally comprises:
i) adding an oligonucleotide tail to the cDNA molecule after step (e), and
ii) optionally, hybridizing the cDNA of (i) with a second synthetic oligonucleotide that comprises a sequence complementary to the oligonucleotide tail, and
iii) optionally using the synthetic oligonucleotide of (ii) as a primer for the synthesis of the complimentary DNA strand,
wherein in step (d) the cDNA is attached to the functionalized particle through a chemical bond between a chemical group present in the functionalized particle and a chemical group present in the first or second synthetic oligonucleotide.

3. Method, according to any of the previous claims, wherein the steps are performed in the following order: (a), (b), (c), (d), (e); or (a), (b), (c), (e), (d).

4. Method, according to any of the previous claims, wherein step b) further comprises eliminating, inactivating, or preventing the elongation of the unhybridized synthetic oligonucleotide.

5. Method, according to any of the previous claims, wherein the elimination, inactivation, or prevention of the elongation of the unhybridized synthetic oligonucleotide is performed using a method selected from: blocking the 3' end of the oligonucleotide using ddTTP, eliminating biotinylated oligonucleotides using streptavidin-associated particles, introducing a sequence in the oligonucleotide designed to hybridize 3' end of the oligonucleotide in a hairpin structure.

6. Method, according to any of the previous claims, wherein the chemical bond is a covalent bond, preferably a triazole bond.

7. Method, according to any of the previous claims, wherein the chemical group present in the functionalized particle is selected from: an azide group, an alkyne group, a carboxyl group, an amino group, a phenyl azide group and a cyclooctyne group.

8. Method, according to any of the previous claims, wherein the chemical group present in the first or second synthetic oligonucleotide is a chemical group present at the 5' end of the first synthetic oligonucleotide.

9. *In vitro* method for detecting, identifying and/or determining the level of an RNA present in sample obtained from a subject, the method comprising generating cDNA from RNA isolated from sample by following the method of any of the claims 1 to 8 and amplifying or quantifying the cDNA or a complementary strand thereof by using preferably PCR.

10. Method for the generation of a functionalized particle comprising an alkyne group or an azide group, or for the modification of particles comprising a carboxyl group into particles comprising an alkyne group or an azide group, which comprises:
a) Providing particles comprising a carboxyl group,
b) Incubating the particle of step (a) in the presence of a linker comprising an amine group in one end, and an alkyne group or an azide group in the other end.

11. Method, according to any of the previous claims, wherein the functionalized particle is a magnetic particle.

12. A functionalized particle obtained by the method of claims 10 to 11.

13. A particle functionalized with an azide or an alkyne chemical group.

14. Particle, according to claims 12 or 13, wherein the functionalized particle or particle is a magnetic particle.

15. Use of the particle of claims 12 to 14 for carrying out the method of claims 1 to 9.
